# EUROPEAN PATENT APPLICATION

(11) **EP 2 426 107 A1**
(43) Date of publication of application: **07.03.2012**
(21) Application number: 10382219.3
(22) Date of filing: 05.08.2010
(51) Int. Cl.: C07D 211/62, C07D 401/12, A61K 51/04, C07F 13/00, C07H 13/10

(54) **Transition metal conjugated radiopharmaceuticals useful as therapeutic and/or diagnostic agents**

(71) Applicant: Universitat Autònoma De Barcelona, 08193 Bellaterra (Barcelona) (ES)
(72) Inventor: Suades I Ortuno, Joan, 08211, CASTELLAR DEL VALLÈS (Barcelona) (ES); Lecina I Vicente, Joan, 08170, MONTORNÈS DEL VALLÈS (Barcelona) (ES); Carrer, Alessia, 30173, MESTRE (VE) (Italia) (IT); Mazzi, Ulderico, 37137, VERONA (Italy) (IT)
(74) Representative: ZBM Patents

(57) **Abstract**

It is provided new compounds, succinimidyl ester of the Zn(II) dithiocarbamate of isonipecotic acid and succinimidyl ester of the Zn(II) dithiocarbamate of nipecotic acid; their preparation processes by reacting any salt of the above compounds with TSTU; conjugated compounds of the above ones, and their preparation processes comprising reacting the succinimidyl ester of the Zn(II) dithiocarbamate of isonipecotic acid or the succinimidyl ester of the Zn(II) dithiocarbamate of nipecotic acid with a molecule which comprises at least a primary and/or secondary amine; and transition metal conjugated compounds, and their preparation processes comprising a transmetallation reaction between the conjugated succinimidyl ester of the Zn(II) dithiocarbamate of isonipecotic acid or the succinimidyl ester of the Zn(II) dithiocarbamate of nipecotic acid with [M(CO)₃(H₂O)₃]X wherein M is a transition metal; and X is a halide anion. The invention further provides the use of the transition metal conjugated compounds as therapeutic and/or diagnostic agents.

## Description

The present invention relates to transition metal conjugated radiopharmaceutical, processes for their preparation, and their use as therapeutic and/or diagnostic agents. It also relates to intermediates useful for their preparation as well as their preparation processes.

### BACKGROUND ART

Radiolabeled compounds are widely used in radiopharmacy for diagnostic and therapeutic purposes, with transition metals as radionuclides, being rhenium (Re) and technetium (Tc) for several reasons of primary interest.

These transition metal centers are applied in two different forms: (1) a highly stable complex, that does not contain a specific (bio)molecule at its backbone and is completely resistant to metabolic degradation (representing the first generation radiopharmaceuticals); and (2) metal complexes attached to a specific (bio)molecule (or biologically active substrate) acting as a biological vector, (representing the second generation of radiopharmaceuticals). Compounds of class (1) are predominantly complexes of Tc. In contrast, most compounds of the second class are represented by halogenated (bio)molecules and less frequently with transition metals such as Tc or Re.

There is an increasing interest in the development of radiopharmaceutical consisting of a transition metal complex attached to a (bio)molecule, also known as transition metal conjugated radiopharmaceuticals, due to lower cost of their preparation and their versatility compared to the preparation of the halogenated ones. The use of a transition metal instead of an halogen in the preparation of the compounds of class (2) has several limitations concerning their preparation, namely that it has to be performed in aqueous solutions; it should yield a high pure compound i.e. no purification step should be necessary, and, finally, the preparation procedure should not exceed a certain time (e.g. about 60 minutes in the case of Tc).

Another limitation of the current methods for synthesising second generation radiopharmaceuticals by direct linking with biomolecules such as peptides is that each biomolecule, i.e. peptide, requires a customised process, i.e. has to be specifically designed.

The above limitations and conditions, and the current state of the art knowledge regarding the use of transition metals for the preparation of radiopharmaceuticals, makes its use rather unattractive.

Consequently there is still a need for new transition metals conjugated radiopharmaceuticals and new processes for their rapid, secure, effective and versatile preparation.

### SUMMARY OF THE INVENTION

The inventors have found new compounds, the succinimidyl ester of the Zn(II) dithiocarbamate of isonipecotic acid of formula (IIIa) and the succinimidyl ester of the Zn(II) dithiocarbamate of nipecotic acid of formula (IIIb), which act as a synton for preparing many different transition metal conjugates. These compounds are advantageous since they allow the provision of a versatile and general platform to prepare a wide range of transition metals conjugated radiopharmaceuticals.

One additional advantage of the use of the succinimidyl ester of the Zn(II) dithiocarbamate of isonipecotic acid and the succinimidyl ester of the Zn(II) dithiocarbamate of nipecotic acid in the preparation of transition metal conjugated radiopharmaceuticals is that the succinimidyl ester of the Zn(II) dithiocarbamate of isonipecotic acid and the succinimidyl ester of the Zn(II) dithiocarbamate of nipecotic acid are very stable compounds that can be stored without changes for several months. Another additional advantage is their very low toxicity. Moreover, a still additional advantage of the succinimidyl ester of the Zn(II) dithiocarbamate of isonipecotic acid and the succinimidyl ester of the Zn(II) dithiocarbamate of nipecotic acid is that they can be prepared by a simple, effective process which provides a very high yield of the compounds, and, furthermore, said compounds can be isolated from the reaction media by simple crystallisation.

Thus, an aspect of the present invention is the provision of the succinimidyl ester of the Zn(II) dithiocarbamate of isonipecotic acid of formula (IIIa) and the succinimidyl ester of the Zn(II) dithiocarbamate of nipecotic acid of formula (IIIb), 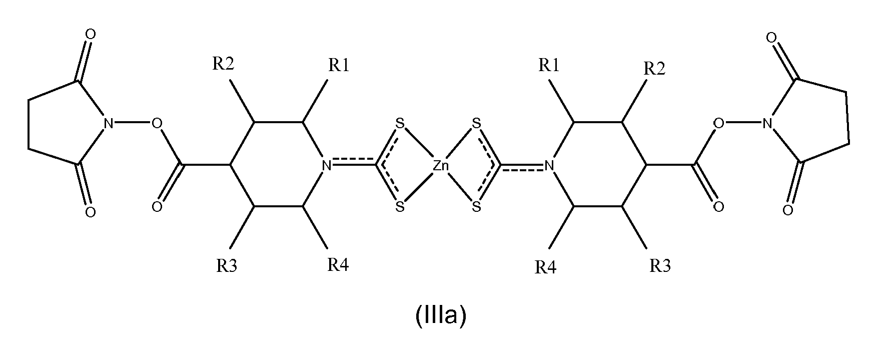 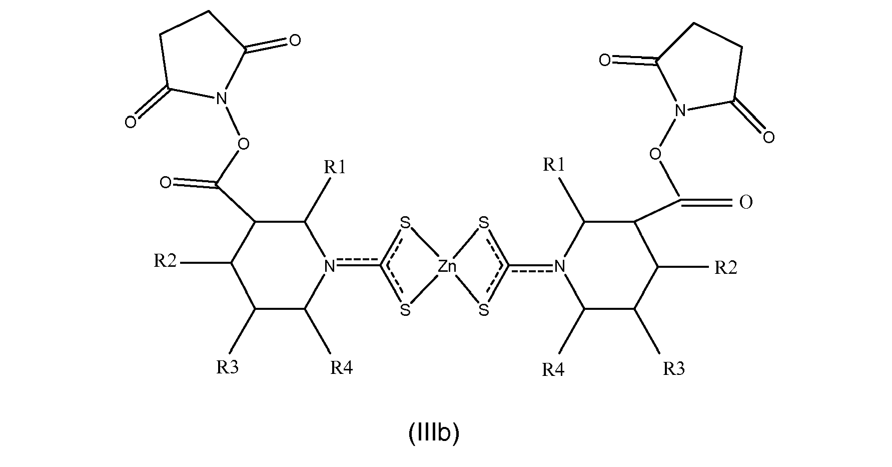 wherein R1, R2, R3 and R4 are independently selected from the group consisting of H, alkyl (C1-C4), alkoxyl (C1-C4), and hydroxyl.

Another aspect of the present invention relates to a preparation process of the succinimidyl ester of the Zn(II) dithiocarbamate of isonipecotic acid of formula (IIIa) or the succinimidyl ester of the Zn(II) dithiocarbamate of nipecotic acid of formula (IIIb), by respectively, reacting a salt of the Zn(II) dithiocarbamate of isonipecotic acid of formula (IVa) or a salt of the Zn(II) dithiocarbamate of nipecotic acid of formula (IVb) 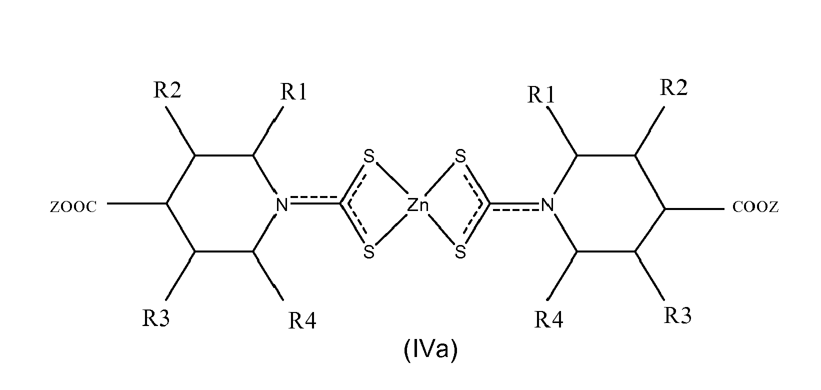 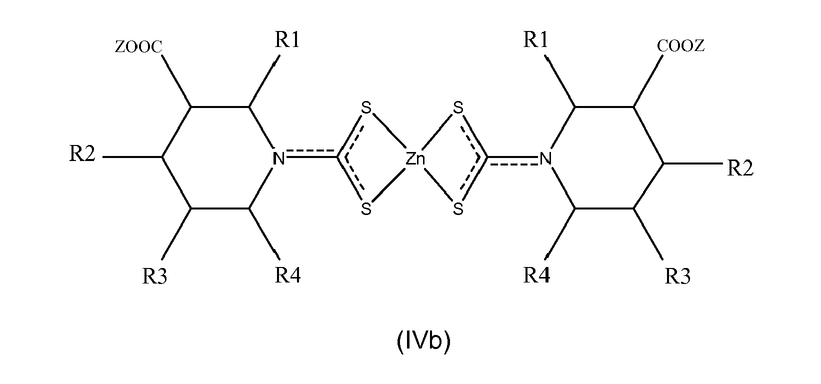 wherein R1, R2, R3 and R4 are independently selected from the group consisting of H, alkyl (C1-C4), alkoxyl (C1-C4), and hydroxyl; and Z is an alkaline metal ion or an organic cation, with N,N,N',N'-tetramethyl-O-(N-succinimidyl)uronium tetrafluoroborate, at room temperature, in the presence of a base.

As mentioned above, the compound of formula (IIIa) and the compound of formula (IIIb) represent a versatile and general platform to prepare transition metal conjugated radiopharmaceuticals, since the compounds of formula (IIIa) and the compound of formula (IIIb) can be used to obtain many different conjugates by reacting it to different molecules through the succinimidyl ester groups of said dithiocarbamates. In other words, a single compound, i.e. the succinimidyl ester of the Zn(II) dithiocarbamate of isonipecotic acid or the succinimidyl ester of the Zn(II) dithiocarbamate of nipecotic acid can be used to prepare a wide range of transition metal conjugated radiopharmaceuticals.

Accordingly, another aspect of the present invention relates to a compound of formula (IIa) or of formula (IIb) 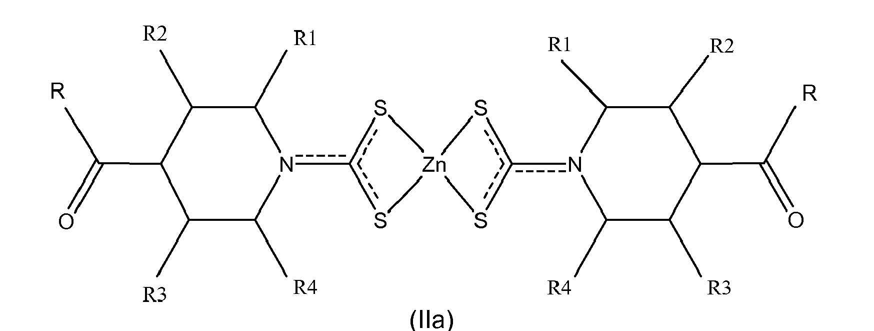 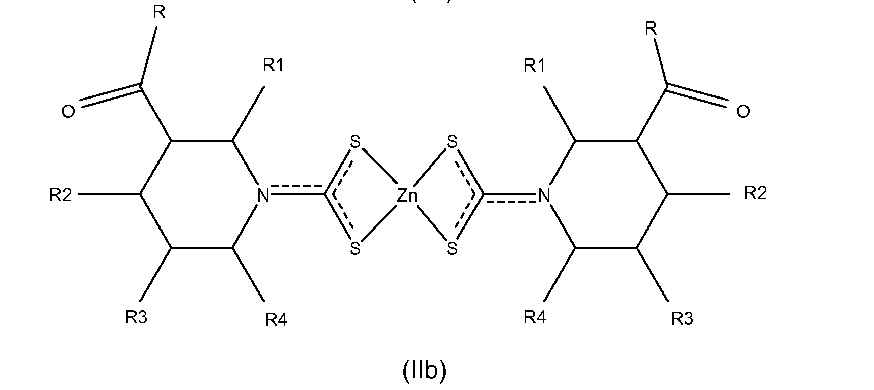 wherein R1, R2, R3 and R4 are independently selected from the group consisting of H, alkyl (C1-C4), alkoxyl (C1-C4), and hydroxyl; R is a radical of a biologically active molecule, which comprises at least a primary and/or secondary amine susceptible to form an amide bond, or an active pharmaceutical ingredient, which comprises at least a primary and/or secondary amine susceptible to form an amide bond. The compound of formula (IIa) and the compound of formula (IIb) are also very stable ones.

Furthermore, another aspect of the present invention relates to the preparation process of the compound of formula (IIa) or the compound of formula (IIb), comprising reacting the succinimidyl ester of the Zn(II) dithiocarbamate of isonipecotic acid of formula (IIIa) or the succinimidyl ester of the Zn(II) dithiocarbamate of nipecotic acid of formula (IIIb), respectively, with a biologically active molecule, which comprises at least a primary and/or secondary amine susceptible to form an amide bond, or an active pharmaceutical ingredient, which comprises at least a primary and/or secondary amine susceptible to form an amide bond, in the presence of a base.

The inventors have also surprisingly found that the compound of formula (IIa) and the compound of formula (IIb) can be used in a transmetallation reaction with a transition metal precursor of formula [M(CO)₃(H₂O)₃]X, wherein M is a transition metal, and X is a halide anion, for the preparation of a compound of formula (Ia) and a compound of formula (Ib), respectively, wherein R1, R2, R3 and R4 are independently selected from the group consisting of H, alkyl (C1-C4), alkoxyl (C1-C4), and hydroxyl; R is a radical of a biologically active molecule, which comprises at least a primary and/or secondary amine susceptible to form an amide bond, or an active pharmaceutical ingredient, which comprises at least a primary and/or secondary amine susceptible to form an amide bond; M is a transition metal; and R' is a monodentate ligand.

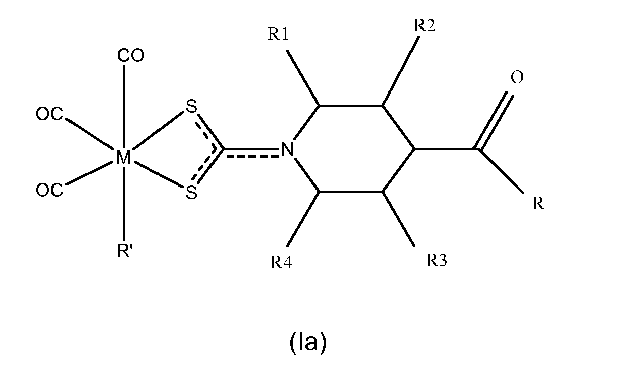

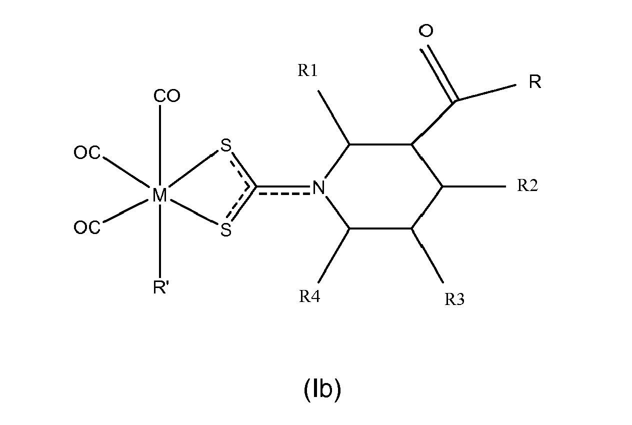

Advantageously, the transmetallation reaction takes place although the solubility of the compounds of formula (IIa) and formula (IIb) in water is very low, and the concentration of the transition metal precursor is also very low.

Consequently, due to the low solubility of the compounds of formula (IIa) and formula (IIb), the preparation process of the present invention has the further advantage of preparing, in an aqueous medium, the compound of formula (Ia) or the compound of formula (Ib), labeled with a transition metal, while the concentrations of the compound of formula (IIa) or of formula (IIb), non-labeled with the transition metal M, is very low. In other words, the preparation process of the present invention allows the preparation of metal transition conjugated radiopharmaceuticals with high ratios between labeled molecule/non-labeled molecule.

Accordingly, another aspect of the present invention relates to the compounds of formula (Ia) and formula (Ib) above.

A further aspect of the present invention encompasses the preparation process of the compound of formula (Ia) or of formula (Ib) comprising a transmetallation reaction between the compound of formula (IIa) or formula (IIb) with a [M(CO)₃(H₂O)₃]X compound, wherein M is a transition metal, and X is a halide anion, in the presence of a monodentate ligand.

The present invention also relates to the use of the compound of formula (IIIa) or formula (IIIb) as defined above for the preparation of a radiopharmaceutical.

The present invention relates to the use of the compound of formula (IIa) or formula (IIb) as defined above for the preparation of a radiopharmaceutical by a transmetallation reaction.

Another aspect of the present invention is the use of the compound of formula (Ia) or formula (Ib) as diagnostic agent.

Another aspect of the present invention relates to the compound of formula (Ia) or formula (Ib) as defined above for use as a medicament.

Still another aspect of the present invention relates to the compound of formula (Ia) or formula (Ib) as defined above for use in the treatment of cancer by means of radiotherapy.

### DETAILED DESCRIPTION OF EMBODIMENTS OF THE INVENTION

The present invention overcomes all the aforementioned limitations.

The term radiopharmaceutical when used in the present invention has the general meaning in the art, i.e. a radioactive pharmaceutical compound used in diagnostics and/or therapy.

As mentioned above, the succinimidyl ester of the Zn(II) dithiocarbamate of isonipecotic acid of formula (IIIa) and the succinimidyl ester of the Zn(II) dithiocarbamate of nipecotic acid of formula (IIIb), have the ability to act as a synton for preparing transition metal conjugated radiopharmaceuticals related to dithiocarbamate-derivatized compounds. In a preferred embodiment, the substituents R1, R2, R3, and R4 of the compound of formula (IIIa) are H. In another preferred embodiment, the substituents R1, R2, R3, and R4 of the compound of formula (IIIb) are H.

The above compounds can be prepared by a process which comprises reacting a salt of the Zn(II) dithiocarbamate of isonipecotic acid of formula (IVa) or a salt of the Zn(II) dithiocarbamate of nipecotic acid of formula (IVb), with N,N,N',N'-tetramethyl-O-(N-succinimidyl)uronium tetrafluoroborate, at room temperature, in the presence of a base.

In a preferred embodiment, the salt of the Zn(II) dithiocarbamate of isonipecotic acid of formula (IVa) and the salt of the Zn(II) dithiocarbamate of nipecotic acid of formula (IVb) used in the above preparation process is a sodium salt. In another preferred embodiment the salt of the Zn(II) dithiocarbamate of isonipecotic acid of formula (IVa) or the Zn(II) dithiocarbamate of nipecotic acid formula (IVb) used in the above preparation process is an ammonium or phosphonium salt.

In a still more preferred embodiment, the above preparation process is performed for the preparation of the compound of formula (IIIa), or the preparation of the compound of formula (IIIb) above, by reacting a salt of the Zn(II) dithiocarbamate of isonipecotic acid of formula (IVa), wherein R1, R2, R3 and R4 are H, or a salt of the Zn(II) dithiocarbamate of nipecotic acid of formula (IVb) wherein R1, R2, R3 and R4 are H. In a still more preferred embodiment, the preparation process is based on the reaction of the sodium salt of the Zn(II) dithiocarbamate of isonipecotic acid of formula (IVa), wherein R1, R2, R3 and R4 are H, or the reaction of the sodium salt of the Zn(II) dithiocarbamate of nipecotic acid of formula (IVb) wherein R1, R2, R3 and R4 are H used.

Preferably, the base is an organic base. More preferably, the base used in the above preparation process is a tertiary amine such as triethylamine, N,N-diisopropylethylamine (DIPEA), or tributylamine; and even more preferably, a tertiary amine with bulky groups. In a particular preferred embodiment the amine is N,N-diisopropylethylamine (DIPEA). Example 1 illustrates a particular embodiment of the above preparation process.

The preparation of a succinimidyl ester from compounds having a carboxylic group is known in the state of the art. However, only Namkung *et al.* (2008) discloses the preparation of a succinimidyl ester from a carboxylate. Moreover, the preparation process disclosed by Namkung *et al.* results in a mixture of the corresponding ester and salt obtained after precipitation with diethyl ether; whereas the preparation process of the present invention results in the pure compound of formula (IIIa) or of formula (IIIb) after precipitation; moreover, the amount of reagent (TSTU) used in the preparation process of the present invention is 10% instead of 20%. The present invention overcomes said difficulties and provides the specific experimental conditions for the effective preparation of the succinimidyl ester of the Zn(II) dithiocarbamate of isonipecotic acid and the succinimidyl ester of the Zn(II) dithiocarbamate of nipecotic acid.

Following the preparation process of the compound of formula (IIIa) or formula (IIIb), can be isolated with a very high yield (≥93%) from the reaction media by simple crystallisation with high purity, above 95%; and, furthermore, since the succinimidyl ester of the Zn(II) dithiocarbamate of isonipecotic acid and the succinimidyl ester of the Zn(II) dithiocarbamate of nipecotic acid are very stable compounds, they can be stored without changes for several months in a fridge.

Interestingly, said compounds of formula (IIIa) or formula (IIIb) can be used to obtain many different bioconjugates of formula (IIa) or formula (IIb) by reacting it to different biologically active molecules or active pharmaceutical ingredients through the succinimidyl ester groups of the dithiocarbamate.

In a preferred embodiment, the substituents R1, R2, R3, and R4 of the compound of formula (IIa) are H, and the substituents R1, R2, R3, and R4 of the compound of formula (IIb) are H; in a still more preferred embodiment, said compounds have as biologically active molecule, 1-(2-methoxyphenyl)piperazine or D-(+)-glucosamine.

As stated above, the biologically active molecule and the active pharmaceutical ingredient can be anyone, from small (<1,000 Da) to large molecules (>15,000 Da), as long as they have at least a primary and/or secondary amine susceptible to form an amide bond. Without limitation, the biologically active molecule could be: biotin, glucose, oestrogen, progesterone, somatostatine, tropane, neuropeptide Y (NPY), bombesin, bitisatin, ScFvs, surfactant protein B, MAb, neurotensine...Again, without limitation, the active pharmaceutical ingredient could be: tamoxifen, WAY-100635, piperidine derivatives, haloperidol, benzazepine, methoxyisobutylisonitrile (MIBI), etomidate ... In a preferred embodiment, the biologically active molecule is 1-(2-methoxyphenyl)piperazine. In another more preferred embodiment, the biologically active molecule is D-(+)-glucosamine.

Conjugates of formula (IIa) or formula (IIb) can be prepared by a process comprising reacting the succinimidyl ester of the Zn(II) dithiocarbamate of isonipecotic acid of formula (IIIa) or the succinimidyl ester of the Zn(II) dithiocarbamate of nipecotic acid of formula (IIIb) with a biologically active molecule, which comprises at least a primary and/or secondary amine susceptible to form an amide group, or an active pharmaceutical ingredient, which comprises at least a primary and/or secondary amine susceptible to form an amide group, in the presence of a base.

The compounds prepared by the above preparation process can be isolated from the reaction media by simple crystallisation with high purity, above 95%. Preferably, the base is an organic base. More preferably, the base used in the above preparation process is a tertiary amine such as triethylamine, N,N-diisopropylethylamine (DIPEA), or tributylamine; and even more preferably, a tertiary amine with bulky groups. In a particular preferred embodiment the amine is N,N-diisopropylethylamine (DIPEA). Example 2 illustrates a particular embodiment of the above preparation process.

Compounds of formula (IIa) or of formula (IIb) can be used in a transmetallation reaction with a transition metal precursor to prepare a compound of formula (Ia) or of formula (Ib), wherein R is a radical of a biologically active molecule, which comprises at least a primary and/or secondary amine susceptible to form an amide group, or of an active pharmaceutical ingredient, which comprises at least a primary and/or secondary amine susceptible to form an amide bond, M is a transition metal, and R' is a monodentate ligand.

In a preferred embodiment, the substituents R1, R2, R3 and R4 of the compound of formula (Ia) are H. In another preferred embodiment, the substituents R1, R2, R3, and R4 of the compound of formula (Ib) are H. In another preferred embodiment, the biologically active molecule is selected from the group consisting of 1-(2-methoxyphenyl)piperazine, and D-(+)-glucosamine.ln another preferred embodiment the transition metal is Re, Tc, or a derived radioisotope. In a more preferred embodiment the transition metal is Re-188/186 or Tc-99m.

It is well known in the art that denticity refers to the number of atoms in a single ligand that binds to a central atom in a coordination complex. When only one atom in the ligand binds to the metal, so the denticity equals one, the ligand is said to be monodentate (also known as unidentate). Ligand refers to an ion or molecule that binds to a central-metal atom to form a coordination complex.

In a preferred embodiment of the present invention, the monodentate ligand is a phosphane (also known as phosphine) or an isonitrile. In a more preferred embodiment, the phosphane is the triphenylphosphine trisulfonate.

In the preparation process of the compound of formula (Ia) or of formula (Ib), the transmetallation occurs between the compound of formula (IIa) or formula (IIb), with the compound [M(COh(H₂O)₃]X, wherein M is a transition metal, and X is an organic or inorganic anion, in the presence of a monodentate ligand.

In a preferred embodiment the transition metal is Re, Tc, or a derived radioisotope. In a more preferred embodiment the transition metal is Re-188/186 or Tc-99m. In a preferred embodiment the anion X is a halide anion. In another preferred embodiment the anion is OTf, also known as triflate anion or trifluoromethanesulfonate anion.

The inventors have surprisingly found that the above transmetallation reaction takes place although the solubility of the Zn(II) dithiocarbamate in water is very low (e.g. no signals are observed in ¹H NMR spectra in D₂O of both the compound of formula (IIa) prepared from the compound of formula (IIIa) and β-alanine methyl ester, and the compound of formula (IIa) prepared from the compound of formula (IIIa) and D-(+)-glucosamine).

In spite of this extremely low solubility, the transmetallation reaction takes place in water at very low transition metal concentration (mass spectroscopy study of the preparation process of compound of formula (I) prepared from the compound of formula (IIa) and β-alanine methyl ester in water medium, shows that the reaction takes place with a concentration of the transition metal precursor as low as 10 ppm).

It is well known in the state of the art that transition metal conjugate radipharmaceuticals comprising bidentate and monodentate ligands, like the compounds of formula (Ia) or formula (Ib) disclosed in the present invention, are useful as diagnostic and therapeutic agents, since the combination of the bi- and monodentate ligands stabilises the transition metal complex and prevents possible binding to serum proteins. One significant advantage of this approach is that the monodentate ligand can be easily modified in order to improve the properties of the drug (e.g. the substitution of triphenylphosphine by triphenyphosphine trisulfonated leads to a more hydrophilic compound).

Consequently, the present invention also relates to the use of the compound of formula (Ia) or formula (Ib) as diagnostic and therapeutic agents.

The diagnostic use of the compound of formula (Ia) or formula (Ib) includes their use in the so-called Single Photon Emission Computerized Tomography (also known as SPECT), as well as Positron Emission Tomography (also known as PET).

The preparation process of the compound of formula (Ia) or formula (Ib) of the present invention can be applied to prepare transition metal bioconjugated radiopharmaceuticals by means of a kit under conditions which can be handled within minutes within a routine clinical environment. The radiopharmaceuticals can then be prepared by the simple addition of a water solution of the metal transition precursor to a solid mixture of the compound of formula (IIa) or formula (IIb) and the monodentate ligand. After mixing the reaction mixture, the residual compound of formula (IIa) or formula (IIb) can be easily separated by centrifugation or filtration.

Accordingly, the present invention relates to a kit for the safe preparation of the compound of formula (Ia) comprising at least the compound of formula (IIa) and a monodentate ligand, both in a single vial or in two separate vials. Similarly, the present invention also encompasses to a kit for the safe preparation of the compound of formula (Ib) comprising at least the compound of formula (IIb) and a monodentate ligand, both in a single vial or in two separate vials.

A compound of formula (Ia) or of formula (Ib) as defined above for use as a medicament, is also part of the invention. In a preferred embodiment, the transition metal of the compound of formula (Ia) or of formula (Ib) for use as a medicament is Re-188, Re-186.

In particular, the compound of formula (Ia) or formula (Ib) as defined above can be used in the radiotherapy. Radiation therapy or radiotherapy is the medical use of ionizing radiation as part of cancer treatment to control malignant cells. Radiotherapy may be used for curative or adjuvant treatment. It is used as palliative treatment (where cure is not possible and the aim is for local disease control or symptomatic relief) or as therapeutic treatment (where the therapy has survival benefit and it can be curative). Furthermore, radiotherapy has several applications in non-malignant conditions, such as the treatment of trigeminal neuralgia, severe thyroid eye disease, pterygium, pigmented villonodular synovitis, prevention of keloid scar growth, and prevention of heterotopic ossification. It is also common to combine radiotherapy with surgery, chemotherapy, hormone therapy or some mixture of the three. Most common cancer types can be treated with radiotherapy in some way. The precise treatment intent (curative, adjuvant, neoadjuvant, therapeutic, or palliative) will depend on the tumor type, location, and stage, as well as the general health of the patient.

Thus, a compound of formula (Ia) or formula (Ib) for use in treatment of cancer by means of radiotherapy, is also part of the invention. This aspect can also be formulated as use of compounds of formula (Ia) or formula (Ib) as defined above for the preparation of a medicament for treatment of cancer by means of radiotherapy in a mammal, including a human.

The invention also relates to a method of treatment by radiotherapy of a mammal, including a human, suffering from cancer, said method comprising the administration to said patient of a therapeutically effective amount of a compound of formula (Ia) or formula (Ib) as defined above, together with pharmaceutically acceptable excipients or carriers

Compounds of the present invention can be used in the same manner as other known radiotherapeutic agents.

Throughout the description and claims the word "comprise" and variations of the word, are not intended to exclude other technical features, additives, components, or steps. Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### EXAMPLES

### Example 1. Preparation of compound of formula (IIIa).

TSTU (256 mg, 0.85 mmol) was added to a solution of Zn(II) dithiocarbamate of isonipecotic acid (200 mg, 0.39 mmol) and DIPEA (13 µL, 0.07 mmol) in dry dimethylformamide (DMF, 1 mL). The heterogeneous mixture was stirred for 16 h and then 140 mL of a mixture H₂O:EtOH (1:1, v/v) was added. The crude precipitated was centrifugated and washed with EtOH and Et₂O to give a white solid that was recrystallized in hot acetonitrile (239 mg, 93% yield): IR (KBr, cm⁻¹): 1736 (C=O, succinimidyl), 1634 (C=O, ester), 1493(C-N, S₂CN); ESI-MS (positive ion): 688.9 (M+Na⁺); elemental analysis calculated for C₂₂H₂₆N₄O₈S₄Zn: C, 39.60 H, 3.90 N, 8.40%. Found: C, 39.82 H, 3.83 N, 8.61 %. The synthetized product was characterized by the usual spectroscopic methods, and the crystal structure determined by x-ray diffraction analysis.

### Example 2. Preparation of compounds of formula (IIa).

The succinimidyl ester of the Zn(II) dithiocarbamate of isonipecotic acid (1.0 g, 1.5 mmol) was dissolved in dry DMF (10 mL). The selected biomolecule (glycine methyl ester, β-alanine methyl ester, 1-(2-methoxyphenyl)piperazine or D-(+)-glucosamine) (3.8 mmol), and DIPEA (7.5 mmol) was added to the solution and stirred overnight. A mixture of water and EtOH (1:1, v/v) (100 mL) was added and the crude was centrifugated and washed with the same mixture to eliminate the excess of the amine and DIPEA. The product was dried under vacuum. The following compounds were prepared:
Zn(II) dithiocarbamate of isonipecotamide, N-glycyl methyl ester. (678 mg, 74% yield): IR (KBr, cm⁻¹): 3294 (N-H, amide), 1753 (C=O, amide), 1646 (C=O, ester), 1492 (C-N, S₂CN); ESI-MS (positive ion): 637.0 (M+Na⁺); elemental analysis calculated for C₂₀H₃₀ON₄OS₄Zn: C, 38.99 H, 4.91 N, 9.09%. Found: C, 38.57 H, 4.84 N, 8.89%.
Zn(II) dithiocarbamate of isonipecotamide, N-β-alanyl methyl ester. (580 mg, 60% yield): IR (KBr, cm⁻¹): 3271 (N-H, amide), 1746 (C=O, amide), 1640 (C=O, ester), 1492 (C-N, S₂CN); ESI-MS (positive ion): 665.1 (M+Na⁺); elemental analysis calculated for C₂₂H₃₄N₄O₆S₄Zn-H₂O: C, 39.91 H, 5.48 N, 8.46%. Found: C, 40.10 H, 5.33 N, 8.55%.
Zn(II) dithiocarbamate of isonipecotamide, N-4(2-methoxyphenyl)piperazyl. (870 mg, 71 % yield): IR (KBr, cm⁻¹): 1639 (C=O, amide), 1436 (C-N, S₂CN); ESI-MS (positive ion): 845.4 (M+Na⁺); elemental analysis calculated for C₃₆H₄₈N₆O₄S₄Zn: C, 52.50 H, 5.80 N, 10.20%. Found: C, 52.24 H, 5.82 N, 10.01%.
Zn(II) dithiocarbamate of isonipecotamide, N-D-(+)-glucosamyl. (517 mg, 31 % yield): IR (KBr, cm⁻¹): 3411 (O-H, alcohols), 1644 (C=O, amide), 1492 (C-N, S₂CN); ESI-MS (positive ion): 817.2 (M+Na⁺); elemental analysis calculated for C₂₆H₄₂N₄O₁₂S₄Zn·2H₂O: C, 37.52 H, 5.57 N, 6.73%. Found: C, 37.88 H, 5.42 N, 6.69%.

### Example 3. Preparation of compound of formula (Ia) wherein the R is a radical of β-alanine methyl ester and R' is PPh₃

Ten ppm solution of [Re(CO)₃(H₂O)₃]⁺ [OTf]⁻ was prepared from [Re(CO)₅(OTf)] following previously reported methods (He *et al.,* 2005, page 5429). The compound of formula (II), wherein R is a radical of β-alanine methyl ester (1 mg) was added to this solution and the resulting mixture was heated to reflux for 1 h. Next, PPh₃ was added (1 mg) and the resulting mixture heated to reflux for an additional hour. The resulting mixture was filtered and the clear solution was analyzed by HPLC-ESI-MS. A Waters reverse-phase column (XTerra MS C18 3.5 µm) was used and the solvents were water with 0.01 % of formic acid (A) and methanol (B). The HPLC gradient system started at 90% A/10% B, which was maintained for 4 min, rising to 5% A / 95% B in 6 min with a linear gradient. It was maintained for 5 min before returning to initial conditions in 2 min. The flow rate was 0.5 ml/min. HPLC (Retention time): 13.2 min. ESI-MS (positive ion, m/z): 844.9 (M+Na⁺), 816.9 (M-CO+Na⁺).

### REFERENCES CITED IN THE APPLICATION

Namkung et al., "Cell-based fluorescence screen for K+ channels and transporters using an extracellular triazacryptand-based K+ sensor", J. Am. Chem. Soc. 2008, vol. 130: 7794-7795.

He et al., "Re(CO)3 complexes synthesized via an improved preparation of aqueous fac-[Re(CO)3(H2O3)]+ as an aid in assessing 99mTc imaging agents. "Structural characterization and solution behavior of complexes with thioether-bearing amino acids as tridentate ligands", Inorg. Chem. 2005, vol. 44: 5437-5446.

## Claims

1. Succinimidyl ester of the Zn(II) dithiocarbamate of isonipecotic acid of formula (IIIa) or succinimidyl ester of the Zn(II) dithiocarbamate of nipecotic acid of formula (IIIb) 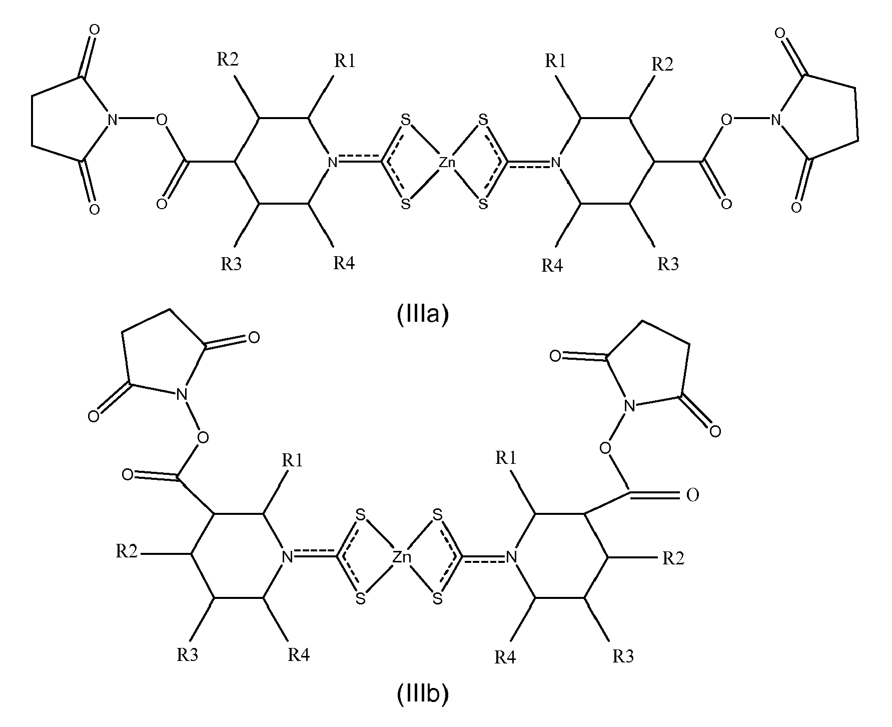 wherein R1, R2, R3, and R4 are independently selected from the group consisting of H, alkyl (C1-C4), alkoxyl (C1-C4), and hydroxyl.

2. The compound according to claim 1, wherein R1, R2, R3, and R4 are H.

3. A preparation process of the succinimidyl ester of the Zn(II) dithiocarbamate of isonipecotic acid of formula (IIIa) or the succinimidyl ester of the Zn(II) dithiocarbamate of nipecotic acid of formula (IIIb) by reacting a salt of the Zn(II) dithiocarbamate of isonipecotic acid of formula (IVa) or a salt of the Zn(II) dithiocarbamate of nipecotic acid of formula (IVb) 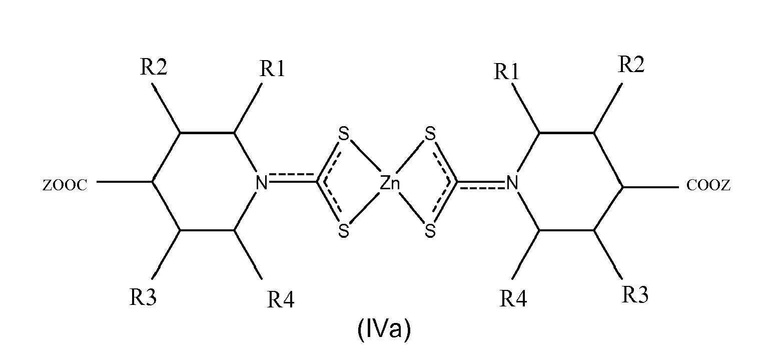 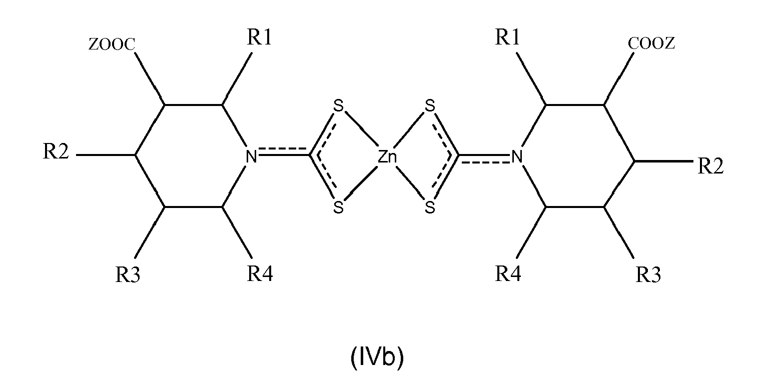 wherein R1, R2, R3 or R4 is independently selected from the group consisting of H, alkyl (C1-C4), alkoxyl (C1-C4), and hydroxyl, and Z is an alkaline metal ion or an organic cation,
with N,N,N',N'-tetramethyl-O-(N-succinimidyl)uronium tetrafluoroborate, at room temperature, in the presence of a base.

4. A compound according to formula (IIa) or formula (IIb), 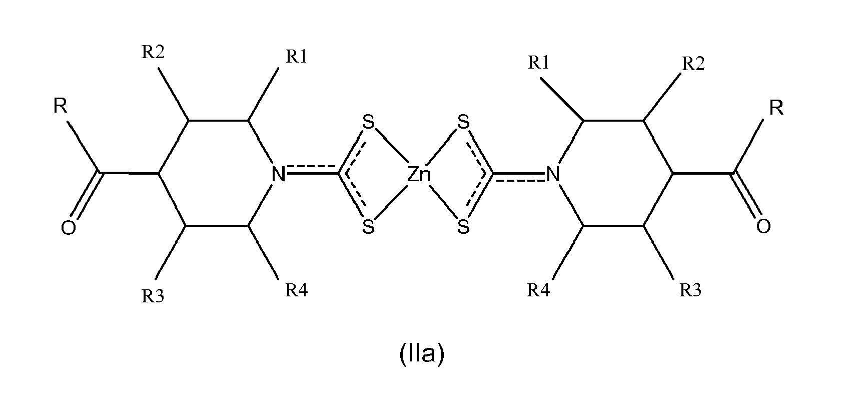 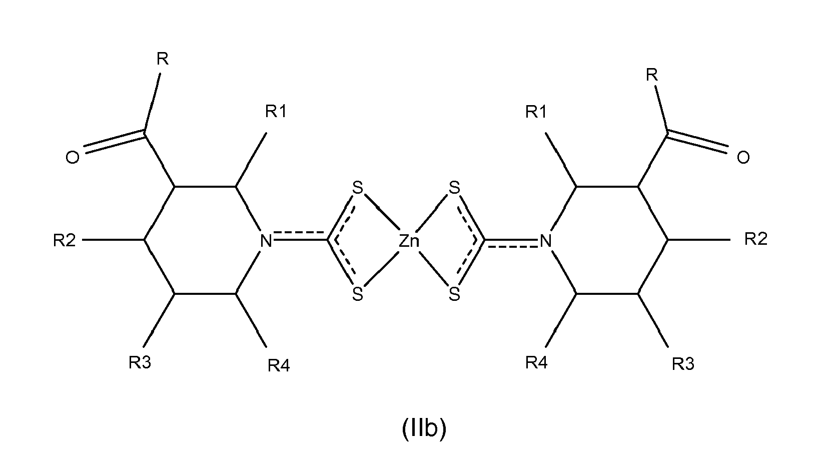 wherein R1, R2, R3, and R4 are independently selected from the group consisting of H, alkyl (C1-C4), alkoxyl (C1-C4), and hydroxyl;
R is a radical of a biologically active molecule, which comprises at least a primary and/or secondary amine susceptible to form an amide bond, or an active pharmaceutical ingredient, which comprises at least a primary and/or secondary amine susceptible to form an amide bond.

5. The compound according to claim 4, wherein R1, R2, R3, and R4 are H.

6. The compound according to any of the claims 4-5, wherein the biologically active molecule is 1-(2-methoxyphenyl)piperazine, or D-(+)-glucosamine

7. A preparation process of the compound of formula (IIa) or formula (IIb) as defined in any of the claims 4-6, comprising reacting the succinimidyl ester of the Zn(II) dithiocarbamate of isonipecotic acid of formula (IIIa) or the succinimidyl ester of the Zn(II) dithiocarbamate of nipecotic acid of formula (IIIb) with a biologically active molecule, which comprises at least a primary and/or secondary amine susceptible to form an amide bond, or an active pharmaceutical ingredient, which comprises at least a primary and/or secondary amine susceptible to form an amide bond, in the presence of a base.

8. A compound of formula (Ia) or formula (Ib), 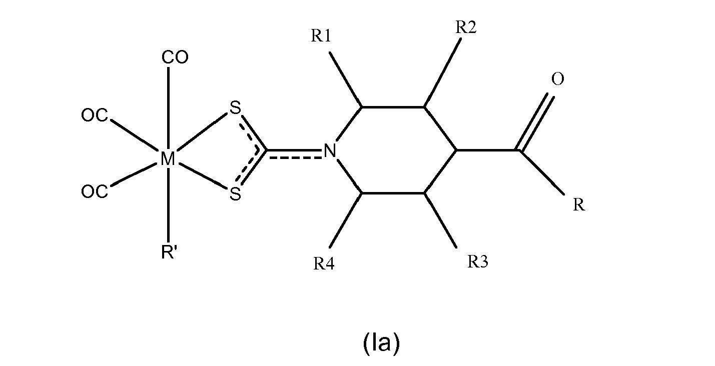 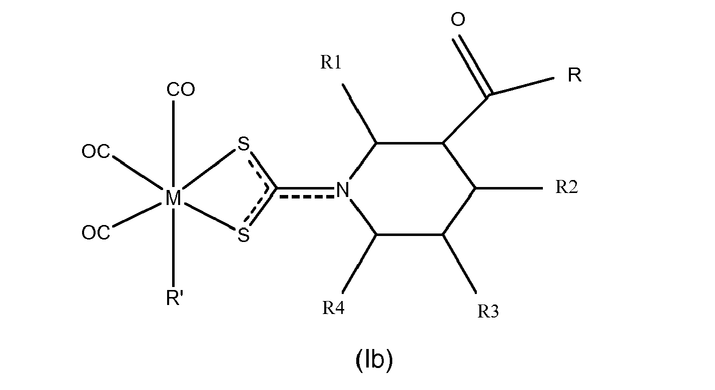 wherein R1, R2, R3, and R4 are independently selected from the group consisting of H, alkyl (C1-C4) and alkoxyl (C1-C4), and hydroxyl; R is a radical of a biologically active molecule, which comprises at least a primary and/or secondary amine susceptible to form an amide bond, or an active pharmaceutical ingredient, which comprises at least a primary and/or secondary amine susceptible to form an amide bond; M is a transition metal; and R' is a monodentate ligand.

9. The compound according to claim 8, wherein R1, R2, R3 and R4 are H.

10. The compound according to any of the claims 8-9, wherein the biologically active molecule is 1-(2-methoxyphenyl)piperazine, or D-(+)-glucosamine.

11. The compound according to any of the claims 8-10, wherein the monodentate ligand is a phosphane or an isonitrile.

12. The compound according to any of the claims 8-11, wherein the transition metal is rhenium, technetium, a derived radioisotope, Re-188/186 or Tc-99m.

13. A preparation process of a compound of formula (Ia) or formula (Ib) as defined in any of the claims 8-12, comprising a transmetallation reaction between the compound of formula (IIa) or formula (IIb) with a [M(CO)₃(H₂O)₃]X compound, wherein M is a transition metal, and X is a halide anion, in the presence of a monodentate ligand.

14. Use of the compound of formula (IIIa) or formula (IIIb) as defined in any of the claims 1 and 2 for the preparation of a radiopharmaceutical.

15. Use of the compound of formula (IIa) or formula (IIb) as defined in any of the claims 4-6, for the preparation of a radiopharmaceutical by a transmetallation reaction.

16. Use of the compound of formula (Ia) or formula (Ib) as defined in any of the claims 8-12 as diagnostic agent.

17. Compound of formula (Ia) or formula (Ib) as defined in any of the claims 8-12, for use as a medicament.

18. Compound of formula (Ia) or formula (Ib) as defined in any of the claims 8-12, for use in the treatment of cancer by means of radiotherapy.
